# EUROPÄISCHE PATENTSCHRIFT

(11) **EP 0 529 275 B1**
(45) Veröffentlichungstag und Bekanntmachung des Hinweises auf die Patenterteilung: **05.04.1995**
(21) Anmeldenummer: 92112160.4
(22) Anmeldetag: 16.07.1992
(51) Int. Cl.: A61F 2/44, F16F 3/04

(54) **Wirbelknochenersatz**
Vertebral prosthesis
Prothèse vertébrale

(30) Priorität: 27.08.1991 DE 4128332
(43) Veröffentlichungstag der Anmeldung: 03.03.1993
(73) Patentinhaber: MAN Ceramics GmbH, 94453 Deggendorf (DE)
(72) Erfinder: Siebels, Wolfgang, W-8360 Deggendorf (DE)

(56) Entgegenhaltungen:
- WO-A-91/06266
- CH-A- 663 256
- DE-C- 3 922 203
- FR-A- 2 626 463
- US-A- 1 905 498
- US-A- 2 892 624
- US-A- 3 905 403
- US-A- 4 309 777
- US-A- 4 554 914
- US-A- 4 759 769
- US-A- 4 764 172
- US-A- 4 892 545
- US-A- 4 969 888

## Beschreibung

Die Erfindung bezieht sich auf einen Wirbelknochenersatz, bestehend aus im wesentlichen zwei annähernd zylindrischen Teilen, die axial mittels eines Drehmechanismus miteinander verbindbar sind, derart, daß die axiale Gesamtlänge der zusammengesetzten Teile veränderbar ist.

Aus der US 4,554,914 ist ein Wirbelknochenersatz dieser Art bekannt, der aus einem ersten schraubenartigen Element und einem zweiten hohlzylindrischen Element mit Innengewinde besteht. Die ineinandergeschraubten Elemente bilden eine stiftartige Stütze, deren freie Enden spitz zulaufen und in die angrenzenden Wirbelknochen eindringen. Als Wirbelkörperersatz werden zwei derartiger Stifte nebeneinander als Abstandshalter zwischen zwei gesunde Wirbelknochen eingesetzt. Um Scherbewegungen bei der bekannten Ausführung zu vermeiden, werden die angrenzenden Wirbel zusätzlich mit Platten verankert.

Der Erfindung liegt die Aufgabe zugrunde, einen Wirbelknochenersatz der eingangs genannten Art zu entwickeln, der mit einfachen Handhabungen implantierbar ist und keiner zusätzlichen Verankerungsmittel bedarf.

Die Aufgabe wird erfindungsgemäß durch die Merkmale des Anspruches 1 gelöst.

Die wendelförmigen Teile werden mit einem Durchmesser ausgelegt, der sich dem Wirbelknochendurchmesser annähert, wodurch Verkantungen oder Scherbewegungen ausgeschlossen werden.

Die erfindungsgemäße Ausführung hat den weiteren Vorteil, daß sie fertigungstechnisch leicht, sowohl aus Metall als auch Kunststoff oder einem faserverstärkten Kunststoff, hergestellt werden kann.

Gemäß einer Ausgestaltung der Erfindung werden die wendelförmigen Teile aus einem mit Matrixmaterial imprägnierten Faserstrang hergestellt. Vorzugsweise werden Kohlenstoffasern verwendet.

Um die axiale Auflage der zusammengesetzten wendelförmigen Teile zu sichern, ist es vorteilhaft, den Querschnitt des Stranges bzw. Faserstranges rechteckig auszubilden. Bei dieser Ausführung und entsprechender Auslegung des Querschnitts und der Windungssteigung ist es möglich, nach Zusammensetzen der beiden Teile einen Hohlzylinder zu erhalten, der eine durchgehende Wandung mit zylindrischem Innen- und Außenmantel bildet. Die beiden Enden können jeweils so ausgebildet werden, daß sie einen elastischen Bereich bilden, um die Elastizität des Knochens nachzubilden.

Für die axiale Verankerung der ineinandergedrehten, wendelförmigen Teile kann eine Stützhülse vorgesehen werden, die nach dem Zusammenbau des Implantats in den durch die Teile gebildeten Hohlzylinder eingesetzt wird. Diese Funktion kann auch durch einen aushärtbaren Knochenzement erfüllt werden, mit dem der Hohlraum des Implantats gefüllt wird.

Gemäß einer weiteren Ausgestaltung der Erfindung weist mindestens eines der beiden Teile Stützelemente auf, mit denen die beiden Teile im ineinandergedrehten Zustand radial verankert werden. Dieses kann beispielsweise durch eine Nut- und Federausführung oder eine Ringschulter oder dergleichen geschehen. Diese Stützelemente können in einer einfachen Weise entlang des gesamten, wendelförmigen Stranges verlaufen oder nur abschnittsweise vorgesehen sein.

Das erfindungsgemäße Implantat hat den weiteren Vorteil, daß lediglich ein Sortiment der wendelförmigen Teile bezüglich deren Durchmesser auf Vorrat gehalten werden muß. Die Implantathöhe wird durch Ablängen der zusammengesetzten Wendelteile für den jeweiligen Bedarf geschaffen. Die Enden werden abschließend mit Endplatten abgedeckt, die eingeklemmt oder eingeklebt werden und die an ihrer äußeren Fläche mit dem angrenzenden Wirbelknochen zusammenwirkende Verankerungsmittel aufweisen.

Die Erfindung wird anhand der in der Zeichnung schematisch dargestellten Ausführungsbeispiele näher beschrieben. Es zeigen:
- Fig. 1: ein erstes Ausführungsbeispiel,
- Fig. 2 und 3: je ein Detail aus Fig. 1 und
- Fig. 4 a bis c: jeweils weitere Ausführungsbeispiele im Querschnitt.

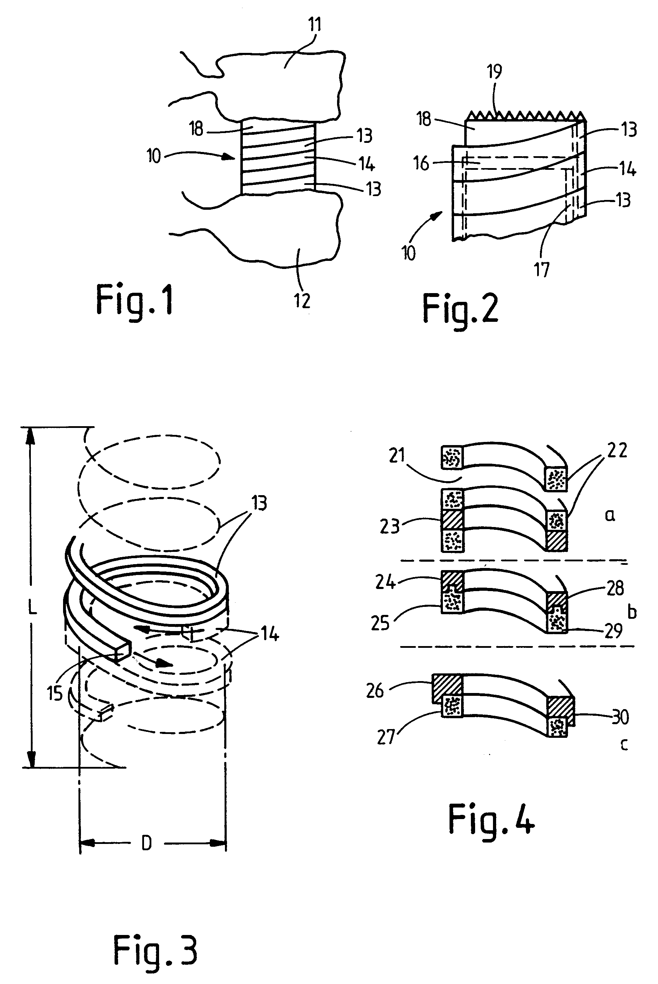

In Fig. 1 ist ein Wirbelknochenersatz 10 gezeigt, der zwischen zwei Wirbelkörper 11 und 12 eingesetzt ist. Das Implantat 10 ist, wie in Fig. 3 näher gezeigt, aus zwei Teilen 13 und 14 gebildet, wobei jedes Teil einen wendelförmig gebogenen Strang 13 bzw. 14 darstellt und wie eine Schraubenfeder aussieht. Beide wendelförmigen Teile 13, 14 haben den gleichen mittleren Durchmesser D. Der Durchmesser richtet sich nach der Dimension der angrenzenden Wirbelkörper 11 und 12. Für die unterschiedlichen Wirbelkörperdimensionen eines sowie unterschiedlicher Patienten wird ein Sortiment von wendelförmigen Teilen mit entsprechenden, unterschiedlichen Durchmessern auf Vorrat zu halten sein.

Zur Bildung eines Wirbelknochenersatzes werden zwei wendelförmige Teile 13, 14 gleichen Durchmessers ineinandergedreht, wie es in Fig. 3 mit den Pfeilen gezeigt ist. Durch entsprechende Auslegung des Querschnittes 15 eines Stranges und der Wendelsteigung liegen die Stränge der beiden Teile 13, 14 annähernd lückenlos ineinander, so daß das Implantat einer zusammengedrückten Schraubenfeder gleicht. Die beiden Teile 13 und 14 werden soweit ineinandergedreht, bis die erforderliche Länge L erreicht ist, worauf die Stränge abgelängt werden. Bei dem so gebildeten Knochenersatz liegen die Stränge 13, 14, wie in Fig. 1 und 2 gezeigt, jeweils aufeinander. Für die radiale Verankerung wird in den Hohlraum 16 des Implantats ein Zylinder 17 eingesetzt. Die Enden bilden jeweils eine Endplatte 18, die nach dem Ablängen der Stränge 13, 14 eingeklemmt, eingesetzt oder eingeklebt werden. Bei der hier beschriebenen Ausführung mit dem eingesetzten Zylinder 17 kann der Zylinder 17 als Anschlag für die Endplatten 18 dienen, so daß weder Einklemm- noch Einklebvorgänge erforderlich sind. Die äußere Stirnseite der Endplatten 18 ist mit Verankerungsmitteln 19, wie z. B. Spitzen, Rauhigkeiten, Dornen versehen, die zur Fixierung des Implantats 10 zwischen den angrenzenden Wirbelkörpern 11 und 12 dienen.

Für die Implantierung werden die beiden entsprechend abgelängten wendelförmigen Teile 13 und 14 vollständig ineinandergedreht bzw. geschraubt, die Endplatten 18 eingesetzt und das Implantat 10 zwischen die Wirbelkörper 11 und 12 gebracht. Anschließend werden die beiden wendelförmigen Teile 13 und 14 zurückgedreht. Letzeres, um das Implantat 10 auf den endgültigen Zwischenabstand zu bringen. Dabei entsteht an den Enden, wie in Fig. 4a gezeigt, jeweils ein Spalt 21 zwischen angrenzenden Strangwindungen. Diese Spalten können zur Einführung von Knochenzement in den Hohlraum 16 des Implantats 10 genutzt werden. Bei der Verwendung von Knochenzement oder Knochenmaterial können die Endplatten 18 auch weggelassen werden. Der Spalt 21 im Implantat kann in Zusammenwirkung mit Knochenzement dem Implantat eine Elastizität einbringen, die der des Knochens entspricht. Eine axiale Elastizität kann auch dadurch erreicht werden, daß der Querschnitt der Stränge mindestens eines der wendelförmigen Teile an den Enden geringer ist, so daß zwischen angrenzenden Wendeln an den Implantatenden Spalte verbleiben. Diese Endbereiche wirken dann als Schraubenfedern.

Der Querschnitt 15 der wendelförmig gebogenen Stränge 13, 14 ist vorzugsweise rechteckig, jedoch ist dessen Konfiguration beliebig wählbar. So können insbesondere Formen oder Vorsprünge gewählt werden, die eine radiale Verankerung der beiden wendelförmigen Teile 13, 14 bewirken.

Fig. 4b und c zeigen zwei Ausführungsbeispiele dazu. Gemäß Fig. 4b besteht das radiale Verankerungsmittel in einer Feder 29 am wendelförmig gebogenen Strang des einen Teiles 25 und einer Nut 28, die im Strang des zweiten Teiles 24 eingebracht ist. Beim Zusammendrehen der beiden Teile 24 und 25 ragt die Feder 29 in die Nut 28 hinein, so daß die beiden wendelförmigen Teile 24 und 25 radial gegeneinander fixiert sind.

Das Verankerungsmmittel kann auch nur in einem Teil 26, wie Fig. 4c zeigt, vorgesehen sein. Dieses Mittel besteht beispielsweise in einer Schulter 30, die am Außenumfang des Stranges des zweiten Teiles 27 angreift.

Die wendelförmigen Teile 13, 14 bzw. 22, 23, 24, 25, 26, 27 können aus für Endoprothesen üblichen Metallen hergestellt werden. Vorzugsweise bestehen sie aus Faserverbundwerkstoff, wobei sowohl die Fasern als auch die Matrix biokompatible Materialen sind. Besonders gut eignen sich Kohlenstoffasern, die imprägniert im Bündel durch eine Form gezogen und im teilgehärtetem Zustand in die Wendelform gebracht und anschließend ausgehärtet werden. Die Form entspricht dem Querschnitt des Faserstranges, wobei die radialen Verankerungselemente 28 bis 30 direkt einbezogen werden können. Auch werden auf diese Weise Endlosstränge zu langen wendelförmigen Teilen geformt, die als Vorrat mit unter- schiedlichen Querschnitten gehalten werden und von denen jeweils nach Bedarf ein Teilstück abgetrennt wird.

## Patentansprüche

1. Wirbelknochenersatz (10), bestehend aus im wesentlichen zwei annähernd zylindrischen Teilen (13, 14; 22, 23; 24, 25; 26, 27), die axial mittels eines Schraubmechanismus miteinander verbindbar sind, derart, daß deren axiale Gesamthöhe veränderbar ist, dadurch gekennzeichnet, daß die beiden Teile (13, 14; 22, 23; 24, 25; 26, 27) jeweils aus einem wendelförmig gebogenen Strang gebildet sind, wobei die beiden wendelförmigen Teile annähernd gleichen Durchmesser (D) haben und unter Bildung eines Hohlzylinders (10) ineinander drehbar sind.

2. Wirbelknochenersatz nach Anspruch 1, dadurch gekennzeichnet, daß die wendelförmigen Teile (13, 14; 22, 23; 24, 25; 26, 27) aus Faserverbundwerkstoff bestehen.

3. Wirbelknochenersatz nach Anspruch 1 oder 2, dadurch gekennzeichnet, daß der Querschnitt (15) des Stranges der wendelförmigen Teile (13, 14) annähernd rechteckig ist.

4. Wirbelknochenersatz nach einem der vorhergehenden Ansprüche, dadurch gekennzeichnet, daß die Wendelform der beiden Teile (13, 14) so ausgelegt ist, daß die beiden Teile im zusammengesetzten Zustand (10) eine annähernd lückenlose Hohlzylinderwand bilden.

5. Wirbelknochenersatz nach einem der vorhergehenden Ansprüche, dadurch gekennzeichnet, daß der Strang mindestens eines der beiden Teile (24 bis 27) Stützelemente (28 bis 30) aufweist, die die beiden ineinandergedrehten, wendelförmigen Teile radial zueinander verankern.

6. Wirbelknochenersatz nach einem der Ansprüche 1 bis 4, dadurch gekennzeichnet, daß innerhalb des aus den beiden wendelförmigen Teilen (13, 14) geformten Hohlzylinders (10) eine Hülse (17) als Radialstütze einsetzbar ist.

7. Wirbelknochenersatz nach einem der vorhergehenden Ansprüche, dadurch gekennzeichnet, daß mit dem angrenzenden Wirbelkörper (11, 12) zusammenwirkbare Endplatten (18) vorgesehen sind, die jeweils an ein freies Ende des zusammengesetzten Knochenersatzes (10) anbringbar sind.

8. Wirbelknochenersatz nach einem der vorhergehenden Ansprüche, dadurch gekennzeichnet, daß Mittel zur Einführung von Knochenzement oder Knochensubstanz in den Hohlraum (16) des hohlzylindrischen Knochenersatzes (10) vogesehen sind.

## Claims

1. A vertebral bone replacement (10), essentially comprising two substantially cylindrical parts (13, 14; 22, 23; 24, 25; 26, 27) axially connectable to each other by means of a screw mechanism in such a manner that their overall axial height is adjustable, characterised in that the two parts (13, 14; 22, 23; 24, 25; 26, 27) are each formed from a helically curved strand, the two helical parts having substantially the same diameter (D) and being rotatable one inside the other to form a hollow cylinder (10).

2. A vertebral bone replacement according to claim 1, characterised in that the helical parts (13, 14; 22, 23; 24, 25; 26, 27) are of fibre composite.

3. A vertebral bone replacement according to claim 1 or 2, characterised in that the cross-section (15) of the strand of the helical parts (13, 14) is substantially rectangular.

4. A vertebral bone replacement according to any one of the preceding claims, characterised in that the helical shape of the two parts (13, 14) is such that the two parts form a substantially gap-free hollow cylinder wall in the assembled state (10).

5. A vertebral bone replacement according to any one of the preceding claims, characterised in that the strand of at least one of the two parts (24 to 27) has supporting members (28 to 30) which anchor the two helical parts, rotated one inside the other, radially in relation to each other.

6. A vertebral bone replacement according to any one of claims 1 to 4, characterised in that a tube (17) can be inserted, as a radial support, inside the hollow cylinder (10) formed from the two helical parts (13, 14).

7. A vertebral bone replacement according to any one of the preceding claims, characterised in that end plates (18) are provided which can co-operate with the adjacent vertebral body (11, 12) and which are each mountable on a free end of the assembled bone replacement (10).

8. A vertebral bone replacement according to any one of the preceding claims, characterised in that means are provided for introducing bone cement or bone matter into the cavity (16) of the hollow cylindrical bone replacement (10).

## Revendications

1. Prothèse de vertèbre (10) constituée essentiellement de deux parties approximativement cylindriques (13, 14 ; 22, 23 ; 24, 25 ; 26, 27) qui sont susceptibles d'être reliées ensemble axialement au moyen d'un mécanisme de vissage de façon que leur hauteur totale axiale soit susceptible d'être modifiée, prothèse de vertèbre caractérisée en ce que les deux parties (13, 14 ; 22, 23 ; 24, 25 ; 26, 27) sont respectivement constituées d'un cordon courbé en forme d'hélice, ces deux parties en forme d'hélice ayant approximativement le même diamètre (D) et étant susceptibles de tourner l'une dans l'autre pour former un cylindre creux (10).

2. Prothèse de vertèbre selon la revendication 1, caractérisée en ce que les parties en forme d'hélice (13, 14 ; 22, 23 ; 24, 25 ; 26, 27) sont constituées d'un matériau composite renforcé par des fibres.

3. Prothèse de vertèbre selon la revendication 1 ou la revendication 2, caractérisée en ce que la section transversale (15) du cordon des parties en forme d'hélice (13, 14) est approximativement rectangulaire.

4. Prothèse de vertèbre selon l'une des précédentes revendications, caractérisée en ce que la forme en hélice des deux parties (13, 14) est conçue de façon que les deux parties à l'état assemblé (10) forment une paroi cylindrique creuse approximativement sans vide.

5. Prothèse de vertèbre selon l'une des précédentes revendications, caractérisée en ce que le cordon d'au moins l'une des deux parties (24 à 27) comporte des éléments de soutien (28 à 30) qui ancrent radialement l'une à l'autre les deux parties en forme d'hélice assemblées l'une dans l'autre.

6. Prothèse de vertèbre selon l'une des revendications 1 à 4, caractérisée en ce qu'à l'intérieur du cylindre creux (10) formé par les deux parties en forme d'hélice (13, 14), une douille (17) jouant le rôle d'appui radial est susceptible d'être mise en place.

7. Prothèse de vertèbre selon l'une des précédentes revendications, caractérisée en ce qu'il est prévu des plaques terminales (18) susceptibles de coopérer avec le corps de vertèbre adjacent (11, 12), ces plaques pouvant être rapportées respectivement à une extrémité libre de la prothèse de vertèbre (10) assemblée.

8. Prothèse de vertèbre selon l'une des précédentes revendications, caractérisée en ce qu'il est prévu des moyens pour l'introduction de ciment d'os ou de substances d'os dans l'espace creux (16) de la prothèse de vertèbre cylindrique creuse (10).
